(19) Europäisches Patentamt
European Patent Office
Office européen des brevets

(11) Numéro de publication: **0 146 443**
**B1**

(12) # FASCICULE DE BREVET EUROPÉEN

(45) Date de publication du fascicule du brevet: **27.04.88**

(51) Int. Cl.⁴: **C 07 C 91/16**, A 61 K 31/135

(21) Numéro de dépôt: **84402313.5**

(22) Date de dépôt: **14.11.84**

(54) **(-)-1-(2-Fluorophényl)-2-tertiobutylamino-1-éthanol, utilisation en thérapeutique.**

(30) Priorité: **15.11.83 FR 8318140**

(43) Date de publication de la demande: **26.06.85 Bulletin 85/26**

(45) Mention de la délivrance du brevet: **27.04.88 Bulletin 88/17**

(84) Etats contractants désignés: **AT BE CH DE GB IT LI LU NL SE**

(56) Documents cité:
**EP-A-0 060 954**
**GB-A-1 043 510**
**GB-A-1 119 125**

(73) Titulaire: **LABORATOIRE L. LAFON Société anonyme dite:, 1 rue Georges Médéric, F-94701 Maisons- Alfort (FR)**

(72) Inventeur: **Lafon, Louis, 5 rue de l'Alboni, F-75016 Paris (FR)**

(74) Mandataire: **Clisci, Serge, S.A. FEDIT- LORIOT CONSEILS EN PROPRIETE INDUSTRIELLE 38, avenue Hoche, F-75008 Paris (FR)**

**EP 0 146 443 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention concerne, en tant que produits industriels nouveaux, le (-)-1-(2-fluorophényl)-2-tertiobutylamino-1-éthanol et ses sels d'addition. Elle concerne également l'utilisation en thérapeutique de ces nouveaux produits.

Dans le brevet EP-A-0 060 954 on a décrit des dérivés appartenant à la famille des N-fluorophénacyl-amines de formule $F-C_6H_4-A-CH_2-NHR$ (où A est CO ou CHOH, et R est isopropyle ou tertiobutyle) et étudié leurs propriétés neuropsychopharmacologiques. Dans EP-A-0 060 954 on a en particulier préconisé le chlorhydrate de (±)-1-(2-fluorophényl)-2-tertiobutylamino-1-éthanol en tant qu'agent antidépresseur du SNC.

On vient de trouver à présent que la base libre lévogyre et ses sels d'addition sont thérapeutiquement plus intéressants que les racémiques et dextrogyres correspondants.

Les nouveaux produits selon l'invention, qui appartiennent à la famille des dérivés de 1-(2-fluorophényl)-2-alkylamino-1-éthanols, sont caractérisés en ce qu'ils sont choisis parmi l'ensemble constitué par

(i) le (-)-1-(2-fluorophényl)-2-tertiobutylamino-1-éthanol ; et,

(ii) ses sels d'addition.

Par sels d'addition, on entend ici, d'une part, les sels d'addition d'acide obtenus par réaction de la base libre avec les acides minéraux et organiques, et, d'autre part, les sels d'ammonium. Parmi les acides utilisables pour salifier la base libre, on peut notamment mentionner les acides chlorhydrique, bromhydrique, acétique, formique, propionique, oxalique, fumarique, maléique, succinique, benzoïque, cinnamique, mandélique, citrique, malique, tartrique, aspartique, glutamique, méthanesulfonique, p-toluènesulfonique. Parmi les composés permettant d'obtenir des sels d'ammonium, on peut notamment citer les halogénures tels que $ICH_3$ et $ClCH_3$.

Le chlorhydrate est le sel d'addition d'acide préféré selon l'invention.

La base libre lévogyre selon l'invention peut être préparée selon une méthode connue per se par application de mécanismes réactionnels classiques. Le procédé que l'on préconise ici, qui est donné dans la préparation I ci-après, comprend la formation du L-(+)-tartrate correspondant, la purification dudit L-(+)-tartrate jusqu'à pouvoir rotatoire constant, puis la séparation de la base libre lévogyre.

Le (-)-1-(2-fluorophényl)-2-tertiobutylamino-1-éthanol et ses sels d'addition, notamment le chlorhydrate, sont utiles en thérapeutique, en particulier en tant qu'agents antidépresseurs.

Selon l'invention on préconise une composition thérapeutique qui est caractérisée en ce qu'elle renferme, en association avec un excipient physiologiquement acceptable, au moins un composé choisi parmi l'ensemble constitué par le (-)-1-(2-fluorophényl)-2-tertio-butylamino-1-éthanol et ses sels d'addition non toxiques, en tant que principe actif.

Bien entendu, dans une telle composition, le principe actif intervient en quantité pharmaceutiquement efficace.

D'autres avantages et caractéristiques de l'invention seront mieux compris à la lecture qui va suivre d'exemples de préparation et de résultats d'essais neuropsychopharmacologiques. L'ensemble de ces éléments n'est pas limitatif mais est donné à titre d'illustration.

**Preparation I**

Obtention du chlorhydrate de (-)-1-(2-fluorophényl)-2-tertio-butylamino-1-éthanol.

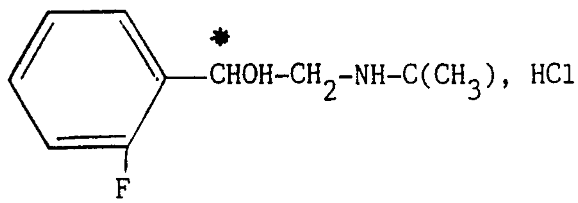

(Exemple 1 ; N° de code : CRL 40 996)

a) On dissout 84,4 g (0,4 mole) du racémique (±)-1-(2-fluorophényl)-2-tertiobutylamino-1-éthanol, dans 1 litre d'isopropanol. On chauffe vers 50° C et ajoute 30 g (0,2 mole) d'acide L-(+)-tartrique. Il se forme un précipité sous agitation. On refroidit, isole le précipité par filtration pour recueillir 56 g de produit renfermant le L-(+)-tartrate.

b) On purifie l'isomère optique en retournant à la base et en précipitant le L-(+)-tartrate selon les modalités opératoires données ci-dessus, trois fois de suite. On isole ainsi 12,5 g (0,0219 mole de L-(+)-tartrate dont le pouvoir rotatoire n'évolue plus.

c) On dissout les 12,5 g dudit tartrate dans 100 ml d'eau et coule la solution limpide ainsi obtenue dans 50 ml de NaOH 1N. On filtre la base précipitée, lave à l'eau et sèche sous vide pour isoler 8,7 g (0,0412 mole) de base pure.

d) On dissout les 8,7 g de ladite base dans 50 ml d'isopropanol, tiédit et acidifie au moyen d'un courant de gaz HCl, précipite sous agitation et refroidit. On filtre et sèche le précipité sous vide pour isoler 8,8 g (0,0355 mole) de CRL 40 996 dont le pouvoir rotatoire (dans le méthanol) à la concentration de 10 g/l est le suivant :

$$[\alpha]_D^{20°C} = -46° \ (c = 10 \text{ g/l} ; CH_3OH)$$

**Préparation II**

Obtention du chorhydrate de (+)-1-(2-fluorphényl)-2-tertio-butylamino-1-éthanol

(Produit CPI ; H° de code: CRL 40 995)

a) le filtrat du stade a) de la préparation I ci-dessus est concentré à sec sous pression réduite. On obtient 58 g de produit renfermant le L-(+)-tartrate de (+)-1-(2-fluorophényl-2-tertiobutylamino-1-éthanol.

b) On retourne à la base libre comme indiqué dans la préparation I et purifie ladite base en précipitant dans l'isopropanol le D-(-)-tartrate. Après trois traitements on isole 16,25 g de D-(-)-tartrate dont le pouvoir rotatoire n'évolue plus.

c) On dissout les 16,25 g dudit tartrate dans 100 ml d'eau et coule la solution limpide résultante dans 50 ml de NaOH 1N. On filtre la base libre précipitée, lave à l'eau et sèche sous vide pour recueillir 11,6 g (0,055 mole) de (+)-1-(2-fluorophényl)-2-tertiobutylamino-1-éthanol pur.

d) On dissout les 11,6 g de base libre dextrogyre dans 50 ml d'isopropanol, tiédit et acidifie au moyen d'un courant de gaz HCl, précipite sous agitation et refroidit. On filtre et sèche le précipité sous vide pour isoler 11 g de CRL 40 995 dont le pouvoir rotatoire est le suivant:

$$[\alpha]_D^{20°C} = +44,75° \ (c = 10g/l \ ; CH_3OH)$$

On a résumé ci-après les résultats des essais qui ont été entrepris avec le chlorhydrate lévogyre (Exemple 1 ; CRL 40 996), le chlorhydrate dextrogyre (CP 1 ; CRL 40 995) et, le cas échéant, le chlorhydrate racémique (CP 2 ; CRL 40 827) antérieurement connu.

Dans ces essais les produits à tester en solution dans de l'eau distillée (pH5) ont été administrés, par voie intrapéritonéale, sous un volume de 20 ml/kg chez la souris mâle, et, sous un volume de 5 ml/kg chez le rat mâle.

## I - Toxicité

Le CRL 40 996 est moins toxique que les CRL 40 995 et CRL 40 827. En effet si la DL-O (dose maximale non mortelle) des trois substances est supérieure ou égale à 64 mg/kg (I.P. ; souris), la DL-60 du CRL 40 996 est de l'ordre de 128 mg/kg (I.P. ; souris) et la DL-100 des CRL 40 995 et CRL 40 827 est inférieure ou égale à 128 mg/kg (I.P. ; souris).

## II - Comportement global et réactivités

Des lots de 6 animaux sont observés avant, puis 0,25 h, 0,50 h, 1 h, 2 h, 3 h, et 24 h après administration des produits à étudier.

On constate que

1. le CRL 40 996 provoque
   a) chez la souris,
   - à la dose de 32 mg/kg :
     - une sédation pendant 0,5 h avec diminution de la réactivité au toucher et du tonus musculaire pendant 0,25 h;
     - une hypothermie (-2,0° C) pendant 1 à 2 h; et,
     - une disparition du "pinna reflex" pendant 0,5 h ;
   - à la dose de 8 mg/kg :
     - une sédation pendant 0,5 h avec diminution de la réactivité au toucher et du tonus musculaire pendant 0,25 h; et,
     - une hypothermie (-1,8° C) pendant 0,25 h;
   - aux doses de 2 et 0,5 mg/kg :
     - un comportement et des réactivités sans modifications sensibles par rapport aux animaux témoins ;

   b) chez le rat,
   - à la dose de 16 mg/kg :
     - une sédation pendant 0,5 h ; et,
     - une dyspnée pendant 1 h ;
   - à la dose de 4 mg/kg :
     - une sédation fugace (0,25 h) ;
   - aux doses de 1 et 0,25 mg/kg :
     - un comportement et des réactivités sans modifications sensibles par rapport aux animaux témoins ;
2. le CRL 40 995 provoque
   a) chez la souris,
   - aux doses de 32 et 8 mg/kg :
     - une respiration déprimée pendant 0,5 h;
   - aux doses de 2 et 0,5 mg/kg :
     - un comportement et des réactivités sans modifications sensibles par rapport aux animaux témoins ;

   b) chez le rat,
   - aux doses de 16 mg/kg, 4 mg/kg, 1 mg/kg et 0,5 mg/kg :
     - un comportement et des réactivités sans modifications sensibles par rapport aux animaux témoins ;

## III - Interaction avec l'apomorphine

Des lots de 6 souris reçoivent chaque produit à tester une demi-heure avant l'injection sous-cutanée de 16 mg/kg d'apomorphine.

Aux doses de 8 mg/kg et 32 mg/kg, le CRL 40 995 s'oppose a l'action hypothermisante de l'apomorphine sans modifier le comportement de verticalisation et les stéréotypies.

En revanche, dès la dose de 0,5 mg/kg, le CRL 40 996 exerce un antagonisme net vis-à-vis de l'hypothermie induite par l'apomorphine sans modifier le comportement de verticalisation et les stéréotypies.

**IV - Interaction avec la réserpine**

Quatre heures après l'injection intrapéritonéale de 2,5 mg/kg de réserpine, des lots de 6 souris reçoivent chaque produit à tester.

Aux doses de 0,5 mg/kg, 2 mg/kg et 8 mg/kg, le CRL 40 995 s'oppose modérément à l'hypothermie réserpinique sans modifier le ptôsis. A dose plus élevée (32 mg/kg), l'antagonisme existe chez 5 souris sur 6 mais est masqué par la perte du réflexe de redressement (et l'hypothermie qui en résulte) chez 1 souris sur 6.

Le CRL 40 996 présente un effet un peu différent. Dès la dose de 0.5 mg/kg, il combat modérément l'hypothermie réserpinique sans modifier le ptôsis. Cet effet qui ne croît pas nettement en intensité avec la dose apparaît avec une latence d'environ 1 heure.

**V - Interaction avec l'oxotrémorine**

Chaque produit à tester est administré à des lots de 6 souris une demi-heure avant l'injection intrapéritonéale de 0,5 mg/kg d'oxotrémorine.

1. Action sur la température.

L'effet hypothermisant de l'oxotrémorine est antagonisé par le CRL 40 996 et le CRL 40 827 mais n'est pas modifié par le CRL 40 995.

2. Action sur les tremblements.

Les tremblements provoqués par l'oxotrémoririe ne sont pas modifiés par les CRL 40 996 et CRL 40 827 ; cependant le CRL 40 995 semble, à la dose de 32 mg/kg, potentialiser ces tremblements.

3. Action sur les symptômes cholinergiques périphériques.

Les CRL 40 995 et CRL 40 827 laissent inchangés les signes de stimulation cholinergique qui apparaissent après l'administration de l'oxotrémorine. Par contre le CRL 40 996 semble diminuer la défécation sans modifier la salivation et la lacrymation.

**VI - Action sur la motilité spontanée**

Une demi-heure après avoir reçu les produits à tester, les souris (6 par dose, 12 témoins) sont placées en actimètre où leur motilité est enregistrée pendant 0,5 h. On observe que les effets des trois produits sont différents

- aux doses de 8 mg/kg et 32 mg/kg, le CRL 40 996 entraîne une diminution très importante de l'activité motrice spontanée de la souris ;
- aux doses de 8 mg/kg et 32 mg/kg, le CRL 40 995 diminue modérément l'activité motrice spontanée ; et,
- à la dose de 8 mg/kg le CRL 40 827 ne modifie pas l'activité motrice spontanée, et, à la dose de 32 mg/kg il semble la diminuer modérément mais pas significativement.

**VII - Action sur le "désespoir comportemental"**

Une demi-heure après avoir reçu les produits à tester, des lots de 6 souris sont placés dans un bécher rempli d'eau sur une hauteur de 6 cm. On note la durée totale d'immobilité entre la 2ème et la 6ème minutes suivant l'immersion.

Le CRL 40 995 ne modifie pas la durée de l'immobilité dite du "désespoir comportemental". En revanche aux doses de 8 et 32 mg/kg, le CRL 40 996 diminue modérément la durée d'immobilité.

**VIII - Conclusion**

Les résultats des essais donnés ci-dessus démontrent que le CRL 40 996 est l'isomère optique actif du CRL 40 827 et présente des effets différents et inattendus par rapport au CRL 40 827 et au CRL 40 995.

En clinique, le CRL 40 996 administré par voie orale chez l'homme s'est avéré être un bon agent antidépresseur dans le traitement des états dépressifs. La posologie préconisée consiste à administrer par jour 3 comprimés ou gélules renfermant chacun 3 à 4 mg de CRL 40 996.

**Revendications** pour les états contractants BE, CH, DE, GB, IT, LI, LU, NI, et SE

1. Nouveau dérivé de 1-(2-fluorophényl)-2-alkylamino-1-éthanol, caractérisé en ce qu'il est choisi parmi l'ensemble constitué par

(i) le (-)-1-(2-fluorophényl)-2-tertiobutylamino-1-éthanol ; et,

(ii) ses sels d'addition.

2. Chlorhydrate de (-)-1-(2-fluorophényl)-2-tertiobutylamino-1-éthanol.

3. Composition thérapeutique, caractérisée en ce qu'elle renferme, en association avec un excipient physiologiquement acceptable, au moins un composé choisi parmi l'ensemble constitué par le (-)-1-(2-fluorophényl)-2-tertiobutylamino-1-éthanol et ses sels d'addition non toxiques selon l'une quelconque des revendications 1 et 2.

**Revendication** pour l'état contractant AT

1. Procédé de préparation de (-)-1-(2-fluorophényl)-2-tertiobutylamino-1-éthanol et de ses sels d'addition, caractérisé en ce qu'il comprend la formation du L-(+)-tartrate correspondant, la purification dudit L-(+)-tartrate jusqu'à pouvoir rotatoire constant, puis la séparation de la base libre lévogyre, puis le cas

écheant la transformation de ladite base libre en sel d'addition.

**Patentansprüche** für die Vertragsstaaten BE, CH, DE, GB, IT, LI, LU, NL, SE

1. Neues 1-(2-Fluorphenyl)-2-alkylamino-1-ethanol, dadurch gekennzeichnet, daß es aus der aus

(i) (-)-1-(2-Fluorphenyl)-2-tert.butylamino-1-ethanol und

(ii) seinen Additionssalzen

gebildeten Gruppe ausgewählt ist.

2. (-)-1-(2-Fluorphenyl)-2-tert.butylamino-1-ethanol. Chlorhydrat.

3. Therapeutische Zusammensetzung, dadurch gekennzeichnet, daß sie in Verbindung mit einem physiologisch annehmbaren Exzipiens zumindest eine aus der aus (-)-1-(2-Fluorphenyl)-2-tert.butylamino-1-ethanol und seinen nichttoxischen Additionssalzen nach einem der Ansprüche 1 und 2 gebildeten Gruppe ausgewählte Verbindung enthält.

**Patentanspruch** für den Vertragsstaat AT

1. Verfahren zur Herstellung von (-)-1-(2-Fluorphenyl)-2-tert.-butylamino-1-ethanol und seiner Additionssalze, dadurch gekennzeichnet, daß es die Bildung des entsprechenden L-(+)-Tartrates, die Reinigung des L-(+)-Tartrates bis zu konstantem Drehvermögen, dann die Abtrennung der freien linksdrehenden Base und dann gegebenenfalls die Umsetzung der freien Base in ein Additionssalz umfaßt.

**Claims** for the contracting States BE, CH, DE, GB, IT, LI, LU, NL, SE

1. Novel 1-(2-fluorophenyl)-2-alkylamino-1-ethanol derivative, characterized in that it is chosen from among the group constituted by

(i) (-)-1-(2-fluorophenyl)-2-t-butylamino-1-ethanol; and

(ii) its addition salts.

2. (-)-1-(2-fluorophenyl)-2-t-butylamino-1-ethanol hydrochloride.

3. Therapeutic composition, characterized in that it contains, in association with a physiologically acceptable excipient, at least one compound chosen from the group constituted by (-)-1-(2-fluoro-phenyl)-2-t-butylamino-1-ethanol and its non-toxic addition salts according to either of the claims 1 and 2.

**Claim** for the contracting State AT

1. Process for the preparation of (-)-1-(2-fluorophenyl)-2-t-butylamino-1- ethanol and its addition salts, characterized in that it comprises the formation of the corresponding L-(+)-tartrate, the purification of said L-(+)-tartrate up to a constant rotatory power, the separation of the levorotatory free base and then, if appropriate, the transformation of said free base into an addition salt.